# FASCICULE DE BREVET EUROPEEN

(11) **EP 2 822 747 B1**
(45) Date de publication et mention de la délivrance du brevet: **18.05.2016**
(21) Numéro de dépôt: 13707688.1
(22) Date de dépôt: 07.03.2013
(51) Int. Cl.: B29C 45/14, A61F 9/02, B29D 11/00, B29C 45/27, B29C 45/00, G02C 7/12, B29L 11/00, A42B 3/22, G02B 5/30, B29K 101/12

(54) **PROCEDE DE REALISATION D'UN ECRAN DE VISION A INSERT SURMOULE PAR INJECTION**
VERFAHREN ZUR ERZEUGUNG EINES BILDSCHIRMS MIT UMSPRITZTEM EINSATZ
METHOD FOR CREATING A VIEWING SCREEN HAVING AN INJECTION OVERMOLDED INSERT

(30) Priorité: 08.03.2012 FR 1200709
(43) Date de publication de la demande: 14.01.2015
(73) Titulaire: BNL Eurolens, 01200 Bellegarde sur Valserine (FR)
(72) Inventeur: CLERC, Didier, F-01200 Eloise (FR); MARTINS, Sébastien, F-01100 Apremont (FR); LEDIEN, Franck, F-01130 Echallon (FR)
(74) Mandataire: Croonenbroek, Thomas Jakob
(86) Numéro de dépôt international: PCT/EP2013/054666
(87) Numéro de publication internationale: WO 2013/132037

(56) Documents cités:
- EP-A1- 1 607 203
- EP-A2- 0 940 244
- US-A1- 2008 231 795

## Description

La présente invention concerne un procédé de réalisation d'un écran de vision à insert surmoulé par injection en particulier pour un écran de vision polarisant, un écran de vision obtenu par ledit procédé ainsi qu'un moule pour la réalisation du procédé.

Plus particulièrement, les écrans de vision à insert surmoulé par injection notamment polarisants obtenues par le procédé de l'invention sont utilisables sur une monture ou un cadre pour former un masque par exemple pour la pratique de certains sports tels que le ski, pour la conduite nocturne, pour un casque de moto ou pour des lunettes de protection.

Les masques polarisants incorporant un filtre polarisant sont très répandus et sont par exemple utilisés dans toutes les activités réalisées dans des endroits, comme la mer ou les étendues neigeuses, comportant des zones de fortes réflexions de la lumière. Ces fortes réflexions peuvent en effet gêner la vision et dans certains cas constituer des sources d'éblouissement. De tels masques ont également un fort intérêt pour la conduite de véhicules, car elles permettent de limiter voire d'éliminer la perception des réflexions parasites sur les parebrises.

Dans ces masques, on utilise un filtre polarisant généralement réalisé sous la forme d'un film de polymère d'alcool polyvinylique (ou PVA). De tels films polarisants sont obtenus classiquement en incorporant des molécules de colorants dichroïques et/ou des cristaux d'iode dichroïques dans un film à base de polymère d'alcool polyvinylique, puis en étirant le film uniaxialement de façon à orienter les molécules de colorants dichroïques et/ou les cristaux d'iode dichroïques selon la direction d'étirement. On entend, par colorant dichroïque, une espèce pouvant être de nature moléculaire ou cristalline et présentant une absorption privilégiée du rayonnement électromagnétique visible pour une orientation spatiale particulière. Les films polarisants ainsi obtenus sont peu onéreux et possèdent une qualité optique qui est compatible avec de nombreuses applications, notamment des applications ophtalmiques.

Le film de PVA est ensuite recouvert d'une ou plusieurs couches de matériau protecteur pour former un insert qui sera surmoulé dans un moule par injection par exemple d'un matériau thermoplastique tel qu'un polycarbonate (PC) ou du polyamide (PA) (comme le nylon) par exemple.

Ainsi, on connaît des inserts composés de TAC - PVA - TAC, (le TAC étant du tri-acétate de cellulose), PC - PVA - PC, TAC - PVA - PC, TAC - PVA - PA, la première couche de l'insert étant celle destinée à être la plus éloignée de l'oeil lors du port du masque et la troisième couche étant celle destinée à être la plus proche de l'oeil de l'utilisateur.

A ce jour, pour la fabrication, on pose l'insert dans le moule et on le maintien mécaniquement par la périphérie à quatre extrémités.

On connaît du document EP 0940244 un procédé de fabrication de lentilles, selon le préambule de la revendication 1, dans lequel plusieurs buses d'aspiration sont utilisées pour maintenir un insert qui sont réparties tout autour du produit fini.

On connaît du document US 2008/23 1795-A un écran de vision comportant trois couches de matiére plastique.

Toutefois, on s'est aperçu que le système de maintien est difficile à régler et peut induire, lors du surmoulage par injection des tensions dans l'insert qui impactent la qualité optique de l'écran de vision.

Un but de l'invention est de fournir un procédé de réalisation d'un écran de vision à insert surmoulé permettant de pallier au moins partiellement aux inconvénients susmentionnés.

A cet effet, on prévoit un procédé de réalisation d'un écran de vision à insert surmoulé par injection, comprenant les étapes de :
- disposer un insert à surmouler dans un moule d'injection,
- maintenir l'insert par au moins une buse d'aspiration intégré dans le moule d'injection,
- injecter dans le moule une matière thermoplastique pour former une couche de support mécanique de l'écran de vision,
- démouler l'écran de vision ainsi formée, caractérisé en ce que le moule possède seulement une buse d'aspiration, qui est disposée dans une zone correspondant pour l'écran de vision à la portion de découpe pour le passage du nez d'un utilisateur.

Selon un aspect, la buse d'aspiration comprend un insert métallique à faible porosité empêchant l'aspiration de la matière thermoplastique injecté et qui est intégré dans la partie concave du moule d'injection.

La buse d'aspiration peut présenter un diamètre transversal compris entre 7,5mm et 10mm, de préférence 9mm.

L'insert métallique de la buse d'aspiration peut présenter une porosité inférieure à 200 µm.

Typiquement, la dépression d'aspiration est comprise entre 0,02 et 0,085 MPa.

Selon un autre aspect, l'insert présente une taille inférieure à celle de l'écran de vision fini.

Une buse d'injection de la matière thermoplastique est disposée de chant par rapport à l'insert, à proximité de la buse d'aspiration et centrée par rapport à celle-ci.

La matière thermoplastique de surmoulage peut être du polycarbonate ou du polyamide.

L'insert peut être formé de trois couches dont la couche intermédiaire est en polymère d'alcool polyvinylique (PVA) et les deux couches externes sont composées pour l'un ou pour les deux de triacétate de cellulose, de polycarbonate ou du polyamide.

L'invention a également pour objet un écran de vision obtenu par le procédé tel que défini ci-dessus comportant, successivement, une couche de support mécanique comprenant une matière thermoplastique formant une face arrière de l'écran de vision, une couche intermédiaire en polymère d'alcool polyvinylique, et une couche externe en matière thermoplastique formant une face avant de l'écran de vision.

En outre, l'invention a pour objet un moule d'injection d'un écran de vision à insert surmoulé, selon les caracateristiques de la revendication 11.

La buse d'injection de la matière thermoplastique est par exemple disposée de chant par rapport à l'insert, à proximité de la buse d'aspiration et centrée par rapport à celle-ci.

D'autres caractéristiques et avantages de l'invention ressortiront à la lecture de la description qui suit d'un mode de réalisation particulier non limitatif de l'invention à la lumière des figures suivantes :
- la figure 1 est une vue schématique en coupe d'un moule,
- la figure 2 est une vue de face d'un écran de vision selon l'invention,
- la figure 3 est une vue en coupe transversale d'un écran de vision selon la figure 2 selon l'axe A-A de la figure 2, et
- la figure 4 est une vue en perspective d'un écran de vision selon la figure 2.

Sur toutes les figures, les mêmes éléments portent les mêmes numéros de référence.

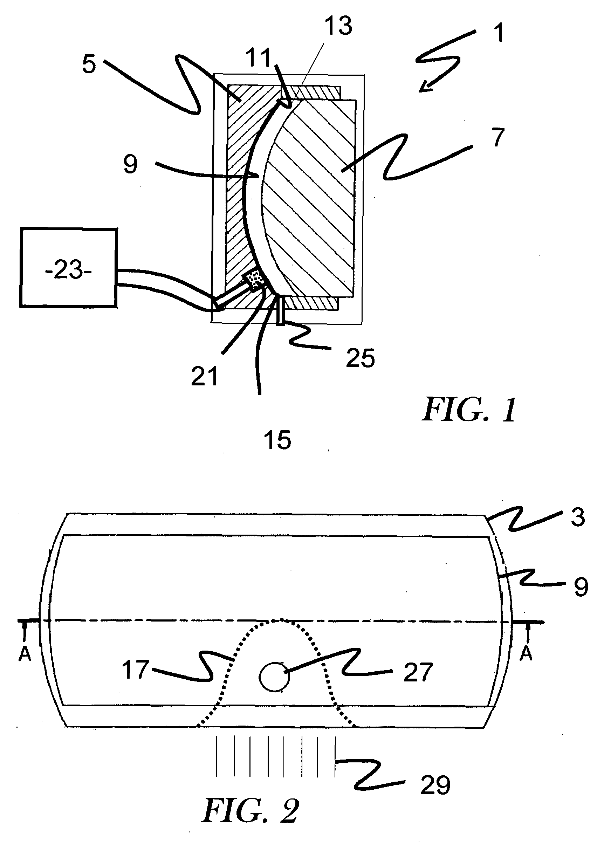

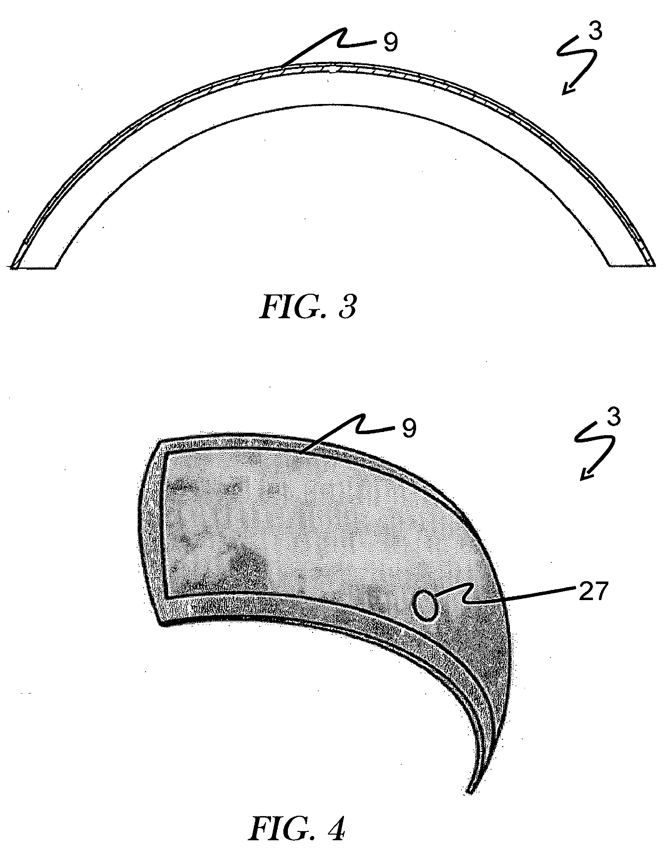

Sur la figure 1 est représenté en coupe transversale un moule 1 d'injection d'un écran de vision 3 (voir figure 4) à insert surmoulé. Ce moule 1 comprend une partie concave 5 et une partie convexe 7.

Comme on le voit dans la figure 1, un insert 9 (trait épais) est disposé dans le moule 1. Cet insert 9 est bombé et ses dimensions sont inférieures aux dimensions de la forme concave 11 de sorte que les bords en haut 13 et en bas 15 du moule (vu sur la figure) ne sont pas en contact avec l'insert 9. L'insert 9 présente donc une taille inférieure à celle de l'écran fini (voir figure 2), ce qui est avantageux car on économise une surface d'insert non négligeable par rapport au procédé de l'état de la technique.

La partie concave 5 présente dans sa partie inférieure (vu sur la figure) et qui correspond pour l'écran de vision à une portion de découpe 17 (voir figure 2) pour le passage du nez d'un utilisateur, une buse d'aspiration 21 intégrée au moule 1 et raccordée à une pompe à vide 23. Lorsque la pompe à vide fonctionne, l'insert 9 est maintenu mécaniquement par aspiration contre la partie concave 5.

Selon l'invention, on a prévu seulement une buse d'aspiration disposée dans la zone 17 correspondant pour l'écran de vision à la portion de découpe pour le passage du nez de l'utilisateur.

La buse d'aspiration comprend par exemple un insert métallique à faible porosité empêchant l'aspiration de la matière thermoplastique injectée et qui est intégré dans la partie concave 5 du moule d'injection 1.

A titre d'exemple, l'insert métallique présente un diamètre transversal compris entre 7,5mm et 10mm, de préférence 9mm.

La porosité de l'insert métallique est inférieure à 200µm, par exemple comprise entre 30 et 100 microns.

L'insert 9 peut être bombé, par exemple par thermoformage, au préalable avant l'insertion dans le moule ou être bombé par thermoformage une fois placé dans le moule et avant l'injection d'une matière thermoplastique.

L'insert est par exemple formé de trois couches dont la couche intermédiaire est en polymère d'alcool polyvinylique (PVA) et les deux couches externes sont composées pour l'un ou pour les deux de triacétate de cellulose (TAC), de polycarbonate (PC) ou du polyamide (PA). On prévoit donc des inserts composés de TAC - PVA - TAC, PC - PVA - PC, TAC - PVA - PC, TAC - PVA - PA, la première couche de l'insert étant celle destinée à être la plus éloignée de l'oeil lors du port du masque et la troisième couche étant celle destinée à être la plus proche de l'oeil de l'utilisateur.

De plus, on voit sur cette figure 1 une buse 25 d'injection d'une matière thermoplastique comme du polycarbonate ou du polyamide (par exemple du nylon), par exemple de qualité « cristal », c'est-à-dire transparente mais non teintée, ou encore teintée / colorée.

Cette buse d'injection 25 de la matière thermoplastique est disposée de chant par rapport à l'insert 9, à proximité de la buse d'aspiration 21 et centrée par rapport à celle-ci.

Le procédé selon l'invention se déroule de la façon suivante :
On dispose un insert 9 à surmouler dans le moule d'injection 1, l'insert ayant été bombée au préalable par thermoformage pour épouser au mieux la paroi de la partie concave du moule 1.

Pour maintenir ensuite l'insert 9 dans le moule 1, on met la pompe à vide 2 3 en marche qui génère un vide et plaque l'insert contre la paroi de la partie concave du moule 1.

A titre d'exemple, la dépression d'aspiration est comprise entre 0,02 et 0,085 MPa.

Puis, on positionne la partie convexe 7 du moule 1 par rapport à de la partie concave 5 en laissant un interstice devant être remplie par de la matière thermoplastique et on injecte dans le moule 1 via la buse 25, une matière thermoplastique comme du polycarbonate ou du polyamide (par exemple du nylon) pour former une couche de support mécanique de l'écran de vision 3.

Enfin, on démoule l'écran de vision 3 ainsi formé.

La figure 2 montre un écran de vision 3 fini qui peut être utilisé comme un masque par exemple pour la pratique de certains sports tels que le ski, pour la conduite nocturne, pour un casque de moto ou pour des lunettes de protection.

On y voit clairement un rond 27 qui est la trace de la buse d'aspiration 21. Toutefois, ceci est sans conséquences, car dans la partie 17, l'écran de vision 3 est découpé pour laisser passer le nez d'un utilisateur.

On voit également sur cette figure 2 des flèches 29 qui symbolisent la buse d'injection de la matière thermoplastique 25. On voit donc que cette buse est centrée par rapport à la buse d'aspiration 21.

L'avantage de cette disposition est que l'on diminue la formation de tensions mécaniques dans l'insert, celles-ci étant concentrées au niveau de la zone 17 qui sera découpée.

La figure 3 montre l'écran de vision 3 en coupe transversale et la figure 4 en perspective.

Bien entendu, la face arrière et la face avant de l'écran de vision peuvent éventuellement être recouvertes par tous revêtements usuellement utilisés dans le domaine des écrans optiques pour apporter une fonction complémentaire. Parmi les revêtements fonctionnels peuvent être disposés sur l'une et /ou l'autre face de l'écran, on peut citer à titre non limitatif les revêtements permettant de conférer en plus une fonction antichoc, une fonction antireflet, une fonction anti-abrasion, une fonction antisalissure, une fonction antibuée, une fonction antistatique ou une combinaison de certaines de ces fonctions.

On comprend donc que le procédé permet de réaliser un écran de vision de qualité évitant en particulier l'altération de la qualité optique de l'écran qui pourrait résulter d'un maintien en plusieurs points périphériques et des tensions surfacique en résultant lors de l'injection de la matière thermoplastique.

En effet, le procédé selon l'invention permet également à l'insert 9 de bien épouser la forme ou l'empreinte de la partie concave 5 du moule 1, ceci sans contrainte. Ceci est important car le thermoformage n'est pas parfaitement sphérique et au même rayon que l'empreinte. Grâce au procédé selon l'invetnion De ce fait il n'y a pas de saccades de la matière chaude lors du remplissage qui peuvent provoqueer des défauts visibles (traits, déformation, etc) avec l'insert qui épouse sans résistance l'empreinte et ceci limite aussi les tensions

Bien entendu, l'invention n'est pas limitée aux modes de réalisation décrits mais englobe toute variante entrant dans le champ de l'invention telle que définie par les revendications.

## Revendications

1. Procédé de réalisation d'un écran de vision (3) à insert (9) surmoulé par injection, comprenant les étapes de :
- disposer un insert (9) à surmouler dans un moule d'injection (1),
- maintenir l'insert (9) par au moins une buse d'aspiration (21) intégré dans le moule d'injection (1),
- injecter dans le moule (1) une matière thermoplastique pour former une couche de support mécanique de l'écran de vision,
- démouler l'écran de vision (3) ainsi formée,
**caractérisé en ce que** le moule présente seulement une buse d'aspiration (21), qui est disposée dans une zone (17) correspondant pour l'écran de vision à la portion de découpe pour le passage du nez d'un utilisateur.

2. Procédé selon la revendication 1, **caractérisé en ce que** la buse d'aspiration (21) comprend un insert métallique à faible porosité empêchant l'aspiration de la matière thermoplastique injectée et qui est intégré dans la partie concave (5) du moule d'injection (1).

3. Procédé selon la revendication 2, **caractérisé en ce que** la buse d'aspiration (21) présente un diamètre transversal compris entre 7,5mm et 10mm, de préférence 9mm.

4. Procédé selon la revendication 2 ou 3, **caractérisé en ce que** l'insert métallique de la buse d'aspiration (21) présente une porosité inférieure à 200 µm.

5. Procédé selon l'une quelconque des revendications précédentes, **caractérisé en ce que** la dépression d'aspiration est comprise entre 0,02 et 0,085 MPa.

6. Procédé selon l'une quelconque des revendications précédentes, **caractérisé en ce que** l'insert (9) présente une taille inférieure à celle de l'écran de vision fini (3).

7. Procédé selon l'une quelconque des revendications précédentes, **caractérisé en ce que** une buse d'injection (25) de la matière thermoplastique est disposée de chant par rapport à l'insert (9), à proximité de la buse d'aspiration (21) et centrée par rapport à celle-ci.

8. Procédé selon l'une quelconque des revendications précédentes, **caractérisé en ce que** la matière thermoplastique de surmoulage est du polycarbonate ou du polyamide.

9. Procédé selon l'une quelconque des revendications précédentes, **caractérisé en ce que** l'insert (9) est formé de trois couches dont la couche intermédiaire est en polymère d'alcool polyvinylique (PVA) et les deux couches externes sont composées pour l'un ou pour les deux de triacétate de cellulose, de polycarbonate ou du polyamide.

10. Ecran de vision (3) obtenu par le procédé selon l'une quelconque des revendications précédentes comportant, successivement, une couche de support mécanique comprenant une matière thermoplastique formant une face arrière de l'écran de vision, une couche intermédiaire en polymère d'alcool polyvinylique, et une couche externe en matière thermoplastique formant une face avant de l'écran de vision.

11. Moule d'injection (1) d'un écran de vision (3) à insert surmoulé, comprenant une partie concave (5) et une partie convexe (7), **caractérisé en ce que** le moule comporte une seule buse d'aspiration (21) permettant de maintenir un insert (9), qui est disposée dans une zone (17) de la partie concave (5) correspondant pour l'écran de vision (3) à la portion de découpe (17) pour le passage du nez de l'utilisateur.

12. Moule d'injection selon la revendication 11, **caractérisé en ce que** la buse d'injection (25) de la matière thermoplastique est disposée de chant par rapport à l'insert (9), à proximité de la buse d'aspiration (21) et centrée par rapport à celle-ci.

## Patentansprüche

1. Verfahren zur Herstellung eines Visiers (3) mit einem Einsatzstück (9), das mittels Spritzguss angegossen wird, wobei es die folgenden Schritte umfasst:
- Bereitstellen eines anzugießenden Einsatzstücks (9) in einer Spritzgießform (1)
- Festhalten des Einsatzstückes (9) durch mindestens eine Ansaugdüse (21), welche in die Spritzgießform (1) integriert ist,
- Einspritzen eines thermoplastischen Stoffs in die Form (1), um eine Schicht zur mechanischen Unterstützung des Visiers zu bilden,
- Entnehmen des auf diese Weise gebildeten Visiers (3) aus der Form,
**dadurch gekennzeichnet, dass** die Form nur eine Ansaugdüse (21) aufweist, die in einem Bereich (17) angeordnet ist, welcher bezüglich des Visiers dem Bereich entspricht, der ausgeschnitten wurde, damit der Benutzer seine Nase hindurchstecken kann.

2. Verfahren nach Anspruch 1, **dadurch gekennzeichnet, dass** die Ansaugdüse (21) ein metallisches Einsatzstück mit geringer Porosität umfasst, welches verhindert, dass der eingespritzte thermoplastische Stoff angesaugt wird, und welches in den konkaven Abschnitt (5) der Spritzgießform (1) integriert ist.

3. Verfahren nach Anspruch 2, **dadurch gekennzeichnet, dass** die Ansaugdüse (21) einen Querdurchmesser im Bereich von 7,5 mm bis 10 mm, vorzugsweise von 9 mm, aufweist.

4. Verfahren nach Anspruch 2 oder 3, **dadurch gekennzeichnet, dass** das metallische Einsatzstück der Ansaugdüse (21) eine Porosität von weniger als 200 µm aufweist.

5. Verfahren nach einem beliebigen der vorhergehenden Ansprüche, **dadurch gekennzeichnet, dass** der Ansaugunterdruck im Bereich von 0,02 bis 0,085 MPa liegt.

6. Verfahren nach einem beliebigen der vorhergehenden Ansprüche, **dadurch gekennzeichnet, dass** das Einsatzstück (9) eine Größe aufweist, die geringer als diejenige des Visier-Endprodukts (3) ist.

7. Verfahren nach einem beliebigen der vorhergehenden Ansprüche, **dadurch gekennzeichnet, dass** eine Düse (25) zum Einspritzen des thermoplastischen Stoffs an der Schmalseite des Einsatzstückes (9) angeordnet ist, in der Nähe der Ansaugdüse (21) und mittig zu dieser.

8. Verfahren nach einem beliebigen der vorhergehenden Ansprüche, **dadurch gekennzeichnet, dass** es sich bei dem thermoplastischen Angießstoff um Polycarbonat oder um Polyamid handelt.

9. Verfahren nach einem beliebigen der vorhergehenden Ansprüche, **dadurch gekennzeichnet, dass** das Einsatzstück (9) aus drei Schichten gebildet ist, wobei die Zwischenschicht aus Polyvinylalkohol(PVA)-Polymer ist und eine der oder beide der zwei Außenschichten sich aus Cellulosetriacetat, Polycarbonat oder Polyamid zusammensetzen.

10. Visier (3), das mittels des Verfahrens nach einem beliebigen der vorhergehenden Ansprüche enthalten wurde, wobei es, nacheinander, eine Schicht zur mechanischen Unterstützung, welche einen thermoplastischen Stoff umfasst, der eine Hinterseite des Visiers bildet, eine Zwischenschicht aus Polyvinylalkoholpolymer und eine Außenschicht aus einem thermoplastischen Stoff, die eine Vorderseite des Visiers bildet, umfasst.

11. Form (1) zum Spritzgießen eines Visiers (3) mit angegossenem Einsatzstück, wobei sie einen konkaven Abschnitt (5) und einen konvexen Abschnitt (7) umfasst, **dadurch gekennzeichnet, dass** die Form eine einzige Ansaugdüse (21) aufweist, die es ermöglicht, ein Einsatzstück (9) festzuhalten, wobei sie in einem Bereich (17) des konkaven Abschnitts (5) angeordnet ist, welcher bezüglich des Visiers (3) dem Bereich (17) entspricht, der ausgeschnitten wurde, damit der Benutzer seine Nase hindurchstecken kann.

12. Spritzgießform nach Anspruch 11, **dadurch gekennzeichnet, dass** die Düse (25) zum Einspritzen des thermoplastischen Stoffs an der Schmalseite des Einsatzstückes (9) angeordnet ist, in der Nähe der Ansaugdüse (21) und mittig zu dieser.

## Claims

1. A method for producing a viewing screen (3) having an injection-overmolded insert (9), comprising the steps of:
- placing an insert (9) that is to be overmolded in an injection mold (1),
- holding the insert (9) using at least one suction nozzle (21) incorporated into the injection mold (1),
- injecting a thermoplastics material into the mold (1) to form a mechanical support layer supporting the viewing screen,
- demolding the viewing screen (3) thus formed,
**characterized in that** the mold has only one suction nozzle (21) positioned in a zone (17) which, insofar as the viewing screen is concerned, corresponds to the cutout portion that accommodates the nose of a user.

2. The method as claimed in claim 1, **characterized in that** the suction nozzle (21) comprises a low-porosity metal insert preventing the injected thermoplastics material from being sucked up and which is incorporated into the concave part (5) of the injection mold (1).

3. The method as claimed in claim 2, **characterized in that** the suction nozzle (21) has a transverse diameter of between 7.5 mm and 10 mm, preferably of 9 mm.

4. The method as claimed in claim 2 or 3, **characterized in that** the metal insert of the suction nozzle (21) has a porosity of below 200 µm.

5. The method as claimed in any one of the preceding claims, **characterized in that** the suction is between 0.02 and 0.085 MPa.

6. The method as claimed in any one of the preceding claims, **characterized in that** the insert (9) has a size smaller than that of the finished viewing screen (3).

7. The method as claimed in any one of the preceding claims, **characterized in that** an injection nozzle (25) for injecting the thermoplastics material is positioned edge-on in relation to the insert (9), near the suction nozzle (21) and centered relative to the latter.

8. The method as claimed in any one of the preceding claims, **characterized in that** the overmolding thermoplastics material is polycarbonate or polyamide.

9. The method as claimed in any one of the preceding claims, **characterized in that** the insert (9) is formed of three layers, the intermediate layer being made of polyvinyl alcohol (PVA) polymer and one or both of the two outer layers being made up of cellulose triacetate, polycarbonate or polyamide.

10. A viewing screen (3) obtained using the method as claimed in any one of the preceding claims, comprising, in succession, a mechanical support layer comprising a thermoplastics material forming a rear face of the viewing screen, an intermediate layer made of polyvinyl alcohol polymer, and an outer layer made of thermoplastics material forming a front face of the viewing screen.

11. An injection mold (1) for molding a viewing screen (3) with an overmolded insert, comprising a concave part (5) and a convex part (7), **characterized in that** the mold comprises just one suction nozzle (21) for holding an insert (9) and positioned in a zone (17) of the concave part (5) which, insofar as the viewing screen (3) is concerned, corresponds to the cutout portion (17) that accommodates the nose of the user.

12. The injection mold as claimed in claim 11, **characterized in that** the thermoplastics-material injection nozzle (25) is positioned edge-on in relation to the insert (9), near the suction nozzle (21) and centered with respect to the latter.
